(19) Europäisches Patentamt
European Patent Office
Office européen des brevets

(11) **EP 1 106 594 B1**

(12) **EUROPÄISCHE PATENTSCHRIFT**

(45) Veröffentlichungstag und Bekanntmachung des Hinweises auf die Patenterteilung:
**05.03.2008 Patentblatt 2008/10**

(51) Int Cl.:
***C07C 45/50*** *(2006.01)*

(21) Anmeldenummer: **00122423.7**

(22) Anmeldetag: **13.10.2000**

(54) **Verfahren zur Hydroformylierung von Olefinen**

Process for the hydroformylation of olefins

Procédé d'hydroformylation d'oléfines

(84) Benannte Vertragsstaaten:
**AT BE CH CY DE DK ES FI FR GB GR IE IT LI LU MC NL PT SE**

(30) Priorität: **30.11.1999 DE 19957528**

(43) Veröffentlichungstag der Anmeldung:
**13.06.2001 Patentblatt 2001/24**

(73) Patentinhaber: **Evonik Oxeno GmbH**
**45772 Marl (DE)**

(72) Erfinder:
• **Wiese, Klaus-Diether, Dr.**
**45721 Haltern (DE)**
• **Protzmann, Guido, Dr.**
**45772 Marl (DE)**
• **Röttger, Dirk, Dr.**
**45657 Recklinghausen (DE)**
• **Büschken, Wilfried, Dr.**
**45721 Haltern (DE)**

(74) Vertreter: **Hirsch, Hans-Ludwig et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
EP-A- 0 987 242          EP-A- 1 057 524
DE-A- 2 538 037          DE-A- 2 715 685
DE-A- 3 234 701          GB-A- 938 831
GB-A- 938 836

**Beschreibung**

Die Erfindung betrifft ein Verfahren zur Herstellung von Aldehyden mit 3-26 Kohlenstoffatomen durch Umsetzung von Olefinen mit 2-25 Kohlenstoffatomen mit Wasserstoff und Kohlenmonoxid in Anwesenheit eines Katalysators in einem Rohrreaktor gemäß Anspruch 1.

Aldehyde werden bei der Synthese vieler organischer Verbindungen eingesetzt. Ihre direkten Folgeprodukte sind Alkohole und Carbonsäuren, die ebenfalls technisch genutzt werden. Durch Aldolkondensation dieser Aldehyde und durch anschließende Hydrierung des Kondensats entstehen Alkohole mit der doppelten Anzahl von Kohlenstoffatomen als die Ausgangsaldehyde. Die aus den Aldehyden durch Hydrierung hergestellten Alkohole finden u.a. Verwendung als Lösungsmittel und als Vorstufe für die Herstellung von Weichmachern und Detergentien.

Es ist bekannt, durch Umsetzung von Olefinen mit Kohlenmonoxid und Wasserstoff Aldehyde und Alkohole (Hydroformylierung, Oxoreaktion) herzustellen. Die Reaktion wird durch Hydridometallcarbonyle, vorzugsweise durch solche von Metallen der 8. Gruppe des Periodensystems, katalysiert. Neben Kobalt, das als Katalysatormetall in großem Umfang technische Anwendung findet, hat in letzter Zeit Rhodium zunehmende Bedeutung erlangt. Im Gegensatz zu Kobalt gestattet Rhodium, die Reaktion bei niedrigen Drücken durchzuführen. Die Hydrierung der Olefine zu gesättigten Kohlenwasserstoffen findet bei Anwendung von RhodiumKatalysatoren in deutlich geringerem Ausmaß statt, als bei Anwendung von Kobalt-Katalysatoren.

Bei den in der Technik durchgeführten Hydroformylierungsverfahren wird der Rhodium-Katalysator während des Prozesses aus einem Katalysatorvorläufer, Synthesegas und gegebenenfalls weiteren Liganden gebildet. Bei Einsatz von modifizierten Katalysatoren können die modifizierenden Liganden im Überschuß im Reaktionsgemisch vorliegen. Besonders bewährt haben sich als Liganden tertiäre Phosphine oder Phosphite. Ihre Anwendung ermöglichte, den Reaktionsdruck auf Werte deutlich unter 300 bar zu senken.

Probleme wirft bei diesem Verfahren jedoch die Abtrennung der Reaktionsprodukte und die Wiedergewinnung der im Reaktionsprodukt homogen gelösten Katalysatoren auf. Im Allgemeinen destilliert man hierzu das Reaktionsprodukt aus dem Reaktionsgemisch ab. In der Praxis kann dieser Weg wegen der thermischen Empfindlichkeit des Katalysators oder der gebildeten Produkte nur bei der Hydroformylierung niedriger Olefine mit bis zu 5 Kohlenstoffatomen im Molekül beschritten werden.

Großtechnisch werden $C_4$- und $C_5$-Aldehyde durch Hydroformylierung beispielsweise nach DE 32 34 701 oder DE 27 15 685 hergestellt.

Im Verfahren nach DE 27 15 685 ist der Katalysator in einer organischen Phase gelöst, die aus Produkt und Hochsieder (entstanden aus dem Produkt) besteht. In dieses Gemisch wird Olefin und Synthesegas eingeleitet. Das Produkt wird mit dem Synthesegas aus dem Reaktor ausgetragen, bzw. als Flüssigkeit abgezogen. Da der Katalysator langsam in seiner Leistung nachläßt, muß ständig ein Teil zusammen mit Hochsieder ausgeschleust und durch eine äquivalente Menge frischen Katalysators ersetzt werden. Wegen des hohen Rhodiumpreises ist die Rückgewinnung des Rhodiums aus dem Ausschleusestrom unerläßlich. Der Aufarbeitungsprozeß ist komplex und belastet somit das Verfahren.

Gemäß DE 32 34 701 wird dieser Nachteil beispielsweise dadurch überwunden, daß der Katalysator in Wasser gelöst ist. Die Wasserlöslichkeit des verwendeten Rhodiumkatalysators wird durch trisulfonierte Triarylphosphine als Liganden erreicht. In die wässrige Katalysatorphase wird Olefin und Synthesegas eingeleitet. Das durch die Reaktion entstehende Produkt bildet eine zweite flüssige Phase. Die flüssigen Phasen werden außerhalb des Reaktors voneinander getrennt und die abgetrennte Katalysatorphase in den Reaktor zurückgeführt.

Das zuletzt genannte Verfahren mit der vorteilhaften Katalysatorabtrennung weist geringere Raum-Zeit-Ausbeuten auf, als Verfahren, bei denen eine flüssige organische Phase mit einem darin gelösten Katalysator vorliegt. Grund dafür ist die unterschiedliche Löslichkeit der Olefine. Während Olefine in einer organischen Lösung gut löslich sind oder sogar selbst die flüssige organische Phase bilden, in der der Katalysator gelöst ist, sind Olefine in wässriger Lösung kaum löslich. Die an für sich schon geringe Löslichkeit der Olefine in einer wässrigen Lösung nimmt mit zunehmender Molmasse der Olefine ab. Dies hat zur Folge, daß höhere Aldehyde nach diesem Verfahren wirtschaftlich nicht hergestellt werden können.

Durch Zusatz eines in der wäßrigen Katalysatorphase löslichen organischen Solvens kann die Reaktionsgeschwindigkeit der Hydroformylierung erhöht werden. Die Verwendung von Alkoholen wie Methanol, Ethanol oder Isopropanol als Cosolvens vergrößert die Reaktionsgeschwindigkeit, hat jedoch den Nachteil, daß Rhodium in die Produktphase übergeht (B. Cornils, W. A Herrmann, Aqueous-Phase Organometallic Catalysis, Wiley-VCH, S. 316-317) und so aus dem Katalysatorkreislauf entfernt wird. Eine Vergrößerung der Reaktionsgeschwindigkeit kann z. B. durch Zusatz von Ethylenglykol erreicht werden. Die Selektivität der Aldehydbildung sinkt jedoch durch diese Maßnahme, da Acetalderivate aus Aldehyd und Ethylenglykol entstehen (V. S. R. Nair, B. M. Bhanage, R.M. Deshpande, R.V. Chaudhari, Recent Advances in Basic and Applied Aspects of Industrial Catalysis, Studies in Surface Science and Catalysis, Vol. 113, 529-539, 1998 Elevier Science B.V.)

In EP 0 157 316 wird die Erhöhung der Reaktiongeschwindigkeit bei der Hydroformylierung von 1-Hexen unter Zusatz von Lösungsvermittlern wie Carbonsäuresalzen, Alkylpolyethylenglycolen oder quaternären Oniumverbindungen beschrieben. Dabei konnte die Produktivität in Abhängigkeit des Lösungsvermittlers um den Faktor 4 gesteigert werden. Die Erhöhung der Reaktionsgeschwindigkeit durch Zusatz von Polyglykolen (z. B. PEG 400) und Polyglykolethern ist bekannt. So wird in DE 197 00 805 C1 die Hydroformylierung von Propen, 1-Buten und 1-Penten und in DE 197 00 804 C1 die Hydroformylierung von höheren Olefinen wie 1-Hexen, 4-Vinylcyclohexen, 1 -Octen, 1-Decen oder 1-Dodecen beschrieben. Diesen Verfahren ist gemeinsam, daß durch den Einsatz von Lösungsvermittlern zwar die Reaktionsgeschwindigkeit erhöht, jedoch die Trennung von wäßriger Katalysatorphase und organischer Produktphase erschwert wird. Dies bedeutet Verluste an Katalysator, der unerwünscht von der wäßrigen in die organische Phase übergeht und Verlust an Wertprodukten, die in der wäßrigen Phase löslich werden. Vermindert man die Menge an Lösungsvermittler, um diese Verluste zu minimieren, so reduziert man gleichzeitig wieder die Reaktionsgeschwindigkeit.

In DE 199 25 384 wird beschrieben, daß die Raum-Zeit-Ausbeute von Aldehyden bei der Hydroformylierung von Olefinen in einer Mehrphasenreaktion, wobei eine kontinuierliche Katalysatorphase und eine weitere flüssige Phase vorliegt, verbessert werden kann, wenn die Umsetzung nicht in einem Rührreaktor, sondern in einem Strömungsreaktor mit einem Belastungsfaktor B > 0,8 durchgeführt wird. Dieses Verfahren zur Hydroformylierung von Olefinen durch Mehrphasenreaktion weist sehr hohe Belastungsfaktoren des Rohrreaktors auf, d. h. eine extrem hohe Durchmischung der Phasen. Der Katalysatorphase können als Zusatz Phasentransfer, oberflächenaktive- oder amphiphile Reagenzien oder Tenside zugesetzt werden, wobei als bevorzugtes Lösungsmittel für den Katalysator Wasser eingesetzt wird.

Der Erfindung lag daher die Aufgabe zugrunde, ein Verfahren zur Hydroformylierung von Olefinen zu entwickeln, das hohe Raum-Zeit-Ausbeuten und Selektivitäten aufweist.

Überraschenderweise wurde gefunden, daß die Hydroformylierung von Olefinen in einer Mehrphasenreaktion unter hohen Ausbeuten und einer niedrigen Bildung von Nebenprodukten durchgeführt werden kann, wenn die Katalysatorphase aus einem Lösungsmittelgemisch besteht.

Gegenstand der vorliegenden Erfindung ist daher ein Verfahren zur Hydroformylierung von einem oder mehreren Olefinen mit 2 bis 25 Kohlenstoffatomen durch Mehrphasenreaktion in einem Rohrreaktor, wobei

a) der Katalysator in der kontinuierlichen Phase enthalten ist,
b) die kontinuierliche Phase ein Lösungsmittelgemisch enthält, gemäß Anspruch 1,
c) mindestens ein Olefin in der dispersen Phase enthalten ist und
d) der Belastungsfaktor des Rohrreaktors gleich oder größer 0.8 ist.

Erfindungsgemäß wird die Hydroformylierung in einem Rohrreaktor, d. h. einem Stömungsrohr durchgeführt. Die Katalysatorphase und die mindestens ein Olefin enthaltende disperse Phase werden in den Rohrreaktor gepumpt. Nach der Reaktion wird das Reaktionsgemisch in eine Produktphase und eine Katalysatorphase getrennt, und die Katalysatorphase in den Rohrreaktor zurückgeführt. Die Produktphase wird aus dem Kreislauf entfernt und kann beispielsweise durch Destillation zur Gewinnung der Aldehyde aufgearbeitet werden.

So können die nach den Verfahren der Erfindung hergestellten Aldehyde zur Herstellung von Alkoholen durch Hydrierung, in Aldolkondensationen oder zur Herstellung von Carbonsäuren durch Oxidation verwendet werden.

Die im erfindungsgemäßen Verfahren verwendete Katalysatorlösung enthält ein Lösungsmittelgemisch und einen Katalysator.

Als eine Komponente des Lösungsmittels können protisch polare Stoffe, wie z.B. Wasser, Ethylenglykol, 1.2-Propylenglykol, 1.3-Propylenglykol, Butandiole oder Glyzerin eingesetzt werden. Eine bevorzugte Lösungsmittelkomponente ist Wasser.

Als weitere Lösungsmittelkomponente zur Bildung des Lösungsmittelgemisches können polare organische Substanzen eingesetzt, insbesondere solche, die mindestens zwei Sauerstoffatome enthalten. Dies sind beispielsweise Verbindungen aus den Stoffklassen der Diole, Triole, Polyole und deren Teil- und Vollether. Beispielhaft seien einige Verbindungen oder Verbindungsgruppen aufgezählt: Ethylenglykol, Ethylenglykolmonoether, Ethylenglykoldiether, Ethylenglykolethoxylate, Ether von Ethylenglykolethoxylaten, Ethylenglykolpropoxylate, Mono- und Diether von Ethylenglykolpropoxylaten, Propylenglykolpropoxylate, deren Mono- und Diether, Polyole die durch Hydrierung von Kohlenhydraten (z.B. hydrierte Monosaccaride, Disaccaride, Oligosaccaride) erzeugt werden, und deren Teil- und Vollether.

Im erfindungsgemäßen Verfahren wird daher als kontinuierliche Phase ein Lösungsmittelgemisch aus Wasser und einem mit Wasser mischbaren organischen Lösungsmittel, enthaltend mindestens zwei Sauerstoffatome, eingesetzt.

Das Massenverhältnis der Lösungsmittel im Lösungsmittelgemisch kann unter Einhaltung folgender Bedingungen in einem weiten Bereich variiert werden:

Das resultierende Gemisch muß eine homogene Phase bilden. Die Löslichkeit für den Katalysator in dieser homogenen Lösung (Phase) muß für die gewünschten Katalysatorkonzentrationen ausreichend sein. Weiterhin darf die Lösung nicht so viskos werden, daß Schwierigkeiten bei der Reaktion und/oder bei der anschließenden Phasentrennung auftreten.

Lösungsmittelgemische werden eingesetzt, die bei 20 °C eine Dielektrizitätskonstante von 50 bis 78 aufweisen.

Beispiele für solche Lösungsmittelgemische sind Wasser/Ethylenglykol-Gemische gemäß der nachfolgenden Tabelle.

| Wasser Gew.-[%] | Ethylenglykol Gew.-[%] | Dielektrizitätskonstante |
| --- | --- | --- |
| 80 | 20 | 74,65 |
| 70 | 30 | 72,19 |
| 60 | 40 | 69,11 |
| 50 | 50 | 65,42 |
| 0 | 100 | 37,89 |

Als Hydroformylierungskatalysatoren können Verbindungen von Metallen der 8. Nebengruppe des Periodensystems der Elemente wie Fe, Co, Ni, Ru, Rh, Pd, Os, Ir oder Pt, bevorzugt in Form von Komplexverbindungen eingesetzt werden. Diese Metallverbindungen sollten zweckmäßig unter den Reaktionsbedingungen in der Katalysatorphase, aber nicht in der Produktphase löslich sein. Werden wäßrige Katalysatorlösungen eingesetzt, erfordert dies wasserlösliche Metallverbindungen. Als bevorzugte Katalysatoren werden Rhodium bzw. wasserlösliche Rhodiumverbindungen eingesetzt. Geeignete Rhodiumsalze sind z. B. Rhodium-III-sulfat, Rhodium-III-nitrat, Rhodium-III-carboxylate wie Rhodiumacetat, Rhodiumpropionat, Rhodiumbutyrat oder Rhodium-2-ethylhexanoat.

Die Art der Liganden für die als Katalysator eingesetzten Metallkomplexe hängt vom verwendeten Metall und dem Lösungsmittelgemisch ab. Diese Komplexe sollten ihre katalytische Wirkung auch im Dauerbetrieb nicht verlieren. Bedingung dafür ist, daß sich die Liganden nicht verändern, wie z.B. durch Reaktion mit dem Lösungsmittel.

Als Liganden für die o. g. katalytisch aktiven Metalle können Triarylphosphine eingesetzt werden. Geeignete Phosphine weisen ein oder zwei Phosphoratome auf, die je Phosphoratom drei Arylreste besitzen, wobei die Arylreste gleich oder verschieden sind und für einen Phenyl-, Naphthyl-, Biphenyl-, Phenylnaphtyl- oder Binaphtyl-Rest stehen. Die Arylreste können mit dem Phosphoratom direkt oder über eine -$(CH_2)x$-Gruppe, worin x für eine ganze Zahl von 1 bis 4, bevorzugt 1 bis 2, besonders bevorzugt 1 steht, verbunden sein. Für wasserlösliche Katalysatorsysteme sollte ein Ligand ein bis drei -$(SO_3)M$ Reste enthalten, worin M gleich oder verschieden ist und für H, ein Alkalimetallion wie Na oder K, ein Ammoniumion, ein quarternäres Ammoniumion, ein (rechnerisch halbes) Erdalkalimetallion wie Ca, oder Mg oder ein Zinkion steht.

Die -$SO_3M$ Reste sind meist Substituenten an den Arylresten und verleihen den Triarylphosphinen die erforderliche Wasserlöslichkeit. Ein bevorzugtes sulfoniertes Triarylphosphin mit einem Phosphoratom ist das Trinatriumtri-(m-sulfophenyl)phosphin.

Anstatt mit Sulfonato-Einheiten (-$SO_3M$) können die verwendeten Phosphine auch mit anderen polaren Gruppen, wie z.B. Carboxylato-Einheiten substituiert sein.

Man kann das Lösungsmittelgemisch unmittelbar, d. h. ohne den Katalysator in der Hydroformylierung einsetzen oder zuvor eine Präformierung des Katalysators im Lösungsmittelgemisch durchführen und das Gemisch mit dem präformierten Katalysator einsetzen. Enthält das Lösungsmittelgemisch Wasser, so läßt sich die Katalysatorlösung jedoch auch auf vergleichsweise einfache Art herstellen, indem man ein wasserlösliches Metallsalz und/oder die wasserlöslichen Liganden in Wasser löst, zur Komplexbildung bringt und anschließend das oder die weiteren Lösungsmittel zur Bildung des Lösungsmittelgemisches zufügt.

Die im erfindungsgemäßen Verfahren verwendete Metallsalzkonzentration kann über einen weiten Bereich eingestellt werden, wobei die Reaktionsgeschwindigkeit auch von der Metallsalzkonzentration abhängt. In der Regel werden bei höheren Metallsalzkonzentrationen höhere Reaktionsgeschwindigkeiten erreicht, andererseits bedeuten höhere Metallsalzkonzentrationen höhere Kosten. Es kann daher je nach Reaktivität des Edukts und der sonstigen Reaktionsbedingungen ein Optimum gewählt werden, das durch orientierende Versuche leicht ermittelt werden kann. Wird Rhodium als aktiver Katalysator eingesetzt, so beträgt der Rhodiumgehalt in der Katalysatorphase üblicherweise 20 ppm bis 2000 ppm, bevorzugt 100 bis 1000 ppm. Das molare Verhältnis zwischen Metall und Liganden kann variiert werden, um das Optimum für jede einzelne Umsetzung zu erreichen. Dieses Verhältnis Metall/Ligand liegt zwischen 1/5 und 1/200, insbesondere zwischen 1/10 und 1/60.

Der pH-Wert der Katalysatorlösung kann für die Hydroformylierung jedes Olefins hinsichtlich der Selektivität der Aldehydbildung optimiert werden. Er liegt zwischen 2 und 8, vorzugsweise zwischen 3,0 und 5,5. Die Einstellung des pH-Wertes kann im laufenden Verfahren durchgeführt werden und z.B. durch Zugabe von Natronlauge oder Schwefelsäure erfolgen.

Als Edukte im erfindungsgemäßen Verfahren können olefinische Verbindungen mit 2-25 Kohlenstoffatomen, bevorzugt mit 3-12 Kohlenstoffatomen eingesetzt werden. Die olefinischen Verbindungen können eine oder mehrere Kohlenstoff-Kohlenstoff-Doppelbindung enthalten, die jeweils endständig oder innenständig angeordnet sein können. Bevorzugt sind olefinische Verbindungen mit endständiger Kohlenstoff-Kohlenstoff-Doppelbindung. Dabei kann ein Olefin einheitlicher Struktur eingesetzt werden. Es können auch Olefingemische eingesetzt werden. Das Gemisch kann aus isomeren Olefinen gleicher Anzahl von Kohlenstoffatomen oder aus Olefinen unterschiedlicher Anzahl von Kohlenstoffatomen oder aus einem Gemisch, das sowohl isomere Olefine als auch Olefine mit unterschiedlicher Anzahl von Kohlenstoffatomen enthält, bestehen. Weiterhin können die Olefine oder Olefingemische unter Reaktionsbedingungen inerte Stoffe wie aliphatische Kohlenwasserstoffe enthalten. Bevorzugt bilden die Olefine, gegebenenfalls mit den inerten Stoffen, die disperse Phase.

Im erfindungsgemäßen Verfahren können Olefine aus den verschiedensten Quellen eingesetzt werden. Beispielsweise seien Olefine aus Crackprozessen, Dehydrierungen oder aus der Fischer-Tropsch-Synthese genannt. Ebenso sind Olefine oder Olefingemische, die durch Dimerisierung, Oligomerisierung, Codimerisierung, Cooligomerisierung oder Metathese von Olefinen entstanden sind, geeignete Edukte.

Die eingesetzten Olefine können (unter Normalbedingungen) gasförmig, flüssig oder fest sein. Feste Olefine werden als Lösungen eingesetzt. Als Lösungsmittel werden inerte, in der Katalysatorphase nicht oder nur wenig lösliche Flüssigkeiten verwendet. Besonders bevorzugt sind Lösungsmittel, die einen höheren Siedepunkt als die herzustellenden Produkte haben, da hierdurch die destillative Abtrennung und Rückführung erleichtert wird.

Bevorzugt werden im erfindungsgemäßen Verfahren α-olefinische Verbindungen eingesetzt. Beispiele für geeignete α-olefinische Verbindungen sind 1-Alkene, Alkylalkenoate, Alkylenalkanoate, Alkenylalkylether und Alkenole, wie z. B. Propen, Buten, Penten, Butadien, Pentadien, 1-Hexen, 1-Hepten, 1-Octen, 1-Nonen, 1-Decen, 1-Undecen, 1-Dodecen, 1-Hexadecen, 2-Ethyl-1-hexen, 1,4-Hexadien, 1,7-Octadien, 3-Cyclohexyl-1-buten, Styrol, 4-Vinylcyclohexen, Allylacetat, Vinylformiat, Vinylacetat, Vinylpropionat, Allylmethylether, Vinylmethylether, Vinylethylether, Allylalkohol, 3-Phenyl-1-propen, Hex-1-en-4-ol, Oct-1-en-4-ol, 3-Butenylacetat, Allylpropionat, Allylbutyrat, n-Propyl-7-octenoat, 7-Octensäure, 5-Hexenamid, 1-Methoxy-2,7-octadien und 3-Methoxy-1,7-octadien. Insbesondere geeignet sind Propen, 1-Buten oder technisch verfügbare Olefingemische, die im wesentlichen 1-Buten, 2-Buten und i-Buten enthalten, und/oder 1-Penten.

Die Produkte der Hydroformylierung von Olefinen sind um ein Kohlenstoffatom längere Aldehyde und gegebenenfalls die entsprechenden Alkohole, die durch eine Hydrierung während des erfindungsgemäßen Verfahrens entstanden sind. Die nach dem erfindungsgemäßen Verfahren hergestellten Aldehyde können aber auch zu den entsprechenden gesättigten Alkoholen hydriert werden, die als Lösungsmittel sowie zur Herstellung von Detergenzien oder Weichmacher verwendet werden können.

Als Hydroformylierungsagens werden im erfindungsgemäßen Verfahren Gemische aus Wasserstoff und Kohlenmonoxid (Synthesegas) oder weitere Gemische aus Wasserstoff, Kohlenmonoxid und unter Reaktionsbedingungen inerten Stoffen eingesetzt. Bevorzugt wird Synthesegas mit 50 Vol.-% $H_2$ und 50 Vol.-% CO verwendet.

Bei Einsatz von flüssigen Olefinen oder festen Olefinen in Lösung ist es günstig, das Hydroformylierungsagens im Überschuß einzusetzen, damit ein möglichst vollständiger Umsatz erreicht wird. Dies senkt den Aufwand für die Aufarbeitung. Bei Einsatz von gasförmigen Olefinen kann es dagegen günstig sein, das Hydroformylierungsreagens im Unterschuß zu verwenden, da das überschüssige gasförmige Olefin sich von der flüssigen Produktphase trennt und wieder in den Prozess zurückgeführt werden kann.

Das molare Verhältnis von Olefin zu Wasserstoff und Olefin zu Kohlenmonoxid kann jeweils größer, kleiner oder gleich 1 sein.

Das erfindungsgemäße Verfahren ist bei Einsatz eines gasförmigen Olefins zunächst eine Zweiphasenreaktion, wobei sich während der Reaktion eine flüssige Produktphase bildet und somit ein Dreiphasensystem entsteht. Bei Einsatz eines flüssigen Olefins liegt von Anfang an mindestens ein Dreiphasensystem vor.

Der im erfindungsgemäßen Verfahren eingesetzte Rohrreaktor kann Füllkörper oder Einbauten enthalten. Füllkörper im Sinne der vorliegenden Erfindung sind beispielsweise: Raschigringe, Sättel, Prallringe, Telleretten, Maschendrahtringe, Maschendrahtgewebe. Beispiele für Einbauten sind Filterplatten, Strombrecher, Kolonnenböden, Lochbleche oder sonstige Mischvorrichtungen. Als Einbauten im Sinne der vorliegenden Erfindung sind aber auch mehrere enge, parallel geschaltete Rohre denkbar, es resultiert also ein Viellrohrreaktor. Besonders bevorzugt sind strukturierte Mischerpackungen oder Demisterpackungen.

Entscheidende Bedeutung hat im erfindungsgemäßen Verfahren die Einhaltung bzw. Überschreitung einer minimalen Querschnittsbelastung bzw. Belastungsfaktor des Rohrreaktors. Bei Aufwärtsbetrieb des Reaktors (Strömungsrichtung von unten nach oben) sollte der Flutpunkt überschritten werden. Der Reaktor wird also oberhalb des Punktes betrieben, bei dem man üblicherweise Blasensäulen betreibt. Bei Abwärtsbetrieb (Strömungsrichtung von oben nach unten) ist die Querschnittsbelastung so einzustellen, daß der Reaktor vollständig geflutet ist. Man arbeitet somit oberhalb des Punktes, bei dem man noch von einer Rieselphase (trickle bed) sprechen kann.

Zur genaueren Festlegung der minimal einzuhaltenden Belastung des Reaktors wird der Belastungsfaktor B des Rohrreaktors als ein dimensionsloser Druckverlust mit

$$B = PD/PS$$

berechnet, wobei PD [Pa/m] ein längenbezogener Druckverlust über den Reaktor unter Betriebsbedingungen und PS [Pa/m] eine Rechengröße mit der Dimension eines längenbezogenen Druckes, definiert als Verhältnis von Massenstrom M [kg/s] aller Komponenten im Reaktor zum Volumenstrom V [m$^3$/s] aller Komponenten unter Betriebsbedingungen, multipliziert mit g = 9,81 m/s$^2$, d. h. PS = (M/V)*g, bedeuten.

Anschaulich wäre PS der statische Druck pro Meter eines Mehrphasengernisches in einem senkrecht stehenden Rohr, wenn alle Phasen mit gleicher Geschwindigkeit strömen würden. PS ist eine reine Rechengröße, die sich aus den dem Reaktor zugeführten Mengenströmen ergibt und die unabhängig von der Strömungsrichtung des Reaktors, der Strömungsgeschwindigkeit aller Phasen oder des Flutzustandes des Reaktors angegeben werden kann.

Der Druckverlust PD [Pa/m] wird als Rechengröße, um die Prozeßbedingungen festzulegen, verwendet und kann nach den gängigen Methoden für Ein- bzw. Mehrphasenströmungen berechnet werden. Gängige Verfahren zur Berechnung des Druckverlustes PD in Rohren, Einbauten oder Füllkörperschüttungen usw. können z. B. im VDI-Wärmeatlas 7. Erweiterte Auflage, VDI-Verlag GmbH, Düsseldorf 1994, Abschnitte Lal bis Lgb7, sowie im Standardwerk Heinz Brauer, Grundlagen der Einphasen- und Mehrphasenströmungen, Verlag Sauerländer, Aarau und Frankfurt am Main, 1971, nachgeschlagen werden.

Der Druckverlust PD ist bei der Einphasenströmung durch ein leeres Rohr durch

$$PD = Cw*\rho/2*w^2/D$$

mit

$\rho$     [kg/m$^3$] Dichte des strömenden Mediums unter Betriebsbedingungen,
w     [m/s] Strömungsgeschwindigkeit (Volumenstrom/Querschnittsfläche),
D     [m] Rohrdurchmesser und
Cw     [-] Widerstandsbeiwert des durchströmten Rohres

gegeben.

Bei einer Strömung durch Füllkörper, Schüttungen oder Einbauten ist die Geschwindigkeit w durch die Effektivgeschwindigkeit (w/$\psi$) sowie der Rohrdurchmesser D durch den hydraulischen Kanaldurchmesser $d_H$ der Füllkörper oder Einbauten zu ersetzen, so daß gilt:

$$PD = Cw*\rho/2*(w/\psi)^2*1/ d_H$$

mit

$d_H$     [m] Hydraulischer Kanaldurchmesser
$\psi$     [-] Leerrohranteil
Cw     [-] Widerstandsbeiwert des durchströmten Apparates mit Füllung

Die füllkörperspezifischen Daten $d_H$ und $\psi$ sind häufig Bestandteil der Lieferspezifikationen von Füllkörpern. Für eine Reihe von Füllkörpern sind Daten im oben erwähnten VDI-Wärmeatlas angegeben.

Der Leerrohranteil $\psi$ kann auch experimentell bestimmt werden, indem man zum Beispiel den Reaktor vor und nach Befüllung mit den Füllkörpern auslitert. Der hydraulische Kanaldurchmesser wiederum kann, wenn er nicht bekannt ist, aus der spezifischen Oberfläche F [m$^2$/m$^3$] der Füllkörper oder Einbauten (in der Regel bekannt oder experimentell bestimmbar) nach der einfachen Beziehung

$$d_H = 4\psi/F.$$

berechnet werden.

Der Widerstandsbeiwert von Rohren, Einbauten und Füllkörpern wird in der Regel in Abhängigkeit von der Reynoldszahl Re beschrieben, die Auskunft über den Strömungszustand unter den gewählten Bedingungen gibt. Bei Füllkörpern, Einbauten usw. ist fast immer folgende Beziehung anwendbar:

$$Cw = K_1/Re^n + K_2/Re^m$$

wobei häufig n = 1, m = 0 (Ansatz nach S. Ergun, Chem. Engng. Progr. 48, (1948), 89), oder n = 1, m = 0,1 (Ansatz nach Brauer et al.) verwendet wird. $K_1$, $K_2$ sind füllkörperspezifische Konstanten, die aus Lieferdaten oder aus der Literatur bekannt sind (Beispiele sind im VDI-Wärmeatlas und bei Brauer et al. zu finden). Sie können aber auch experimentell bestimmt werden, indem man den Rohrreaktor mit Füllkörpern mit einer Flüssigkeit unter verschiedenen Geschwindigkeiten betreibt und aus den bekannten Daten und dem gemessenen Druckverlust Cw in Abhängigkeit von Re bestimmt.

Die dimensionslose Reynoldszahl Re schließlich ist definiert als Re = w*(ρ/η)*D für Leerrohre bzw . Re = (w/ψ) * (ρ/η) *$d_H$ für Rohre mit Einbauten oder Füllkörpern, η [Pa*s] bezeichnet jeweils die Viskosität und ρ [kglm³] die Dichte des strömenden Mediums.

Der Druckverlust bei Zweiphasenströmungen (hier gas-flüssig für Synthesegas/Katalysatorlösung) steigt überproportional an. Meist wird nach Lockhart-Martinelli (in Brauer et al.) der Druckverlust der Zweiphasenströmung $P_{1g}$ auf den Druckverlust einer der beiden Phasen bezogen, zum Beispiel auf den Druckverlust der reinen strömenden flüssigen Phase $P_1$, und in Beziehung zu dem Verhältnis des Druckverlustes der beiden als allein strömend gedachten Phasen $P_1$ und $P_g$ gesetzt.

Zur Berechnung von Druckverlusten in Zweiphasenströmungen werden häufig dimensionslose Drücke nach $\phi^2 = P_{1g}/P_1$ und $X^2 = P_1/P_g$ eingesetzt. Der weitere Zusammenhang $\phi^2$ = Funktion($X^2$) ist vielfach untersucht. Beispiele finden sich in folgenden Literaturstellen:

Y. Sato, T.Hirose, F. Takahashi, M. Toda: "Pressure Loss and Liquid Hold Up in Packed Bed Reactor with Cocurrent Gas-Liquid Down Flow"; J. Chem. Eng. Of Japan, Vol 6 (Nr. 2), 1973, 147-152;
D. Sweeney: "A Correlation for Pressure Drop in Two-Phase Concurrent Flow in Packed Beds"; AIChE-Journal, Vol. 13, 7/1967, 663-669;
V. W. Weekman, J. E. Myers: "Fluid-Flow Characteristics of Concurrent Gas-Liquid Flow in Packed Beds"; AIChE-Journal, Vol 10 (Nr. 6), 11/1964, 951-957;
R. P. Larkins, R P. White, D. W. Jeffrey: "Two-Phase Concurrent Flow in Packed Beds"; AIChE-Journal, Vol 7 (Nr. 2), 6/1961, 231-239 oder
N. Midoux, M. Favier, J.- C. Charpentier: " Flow Pattern, Pressure Loss and Liquid Holdup Data in Gas-Liquid Down-Flow Packed Beds with Foaming and Nonfoaming Liquids"; J. Chem. Eng. Of Japan, Vol 9 (Nr. 5), 1976, 350-356. Häufig wird für die Berechnung der von Midoux vorgeschlagene Zusammenhang, der für viele Gas-Flüssig-Systeme überprüft wurde, benutzt. Bei nichtschäumenden Systemen ist beispielsweise

$$\phi^2 = 1 + 1/X + 1,14/X^{0,54}$$

Dieser nach Lockart-Martinelli benannte Zusammenhang ist in vielen Werken graphisch dargestellt, detaillierte Abhandlungen hierüber finden sich in vielen Lehrbüchern der Verfahrenstechnik und Veröffentlichungen, so auch bei Brauer et al.

Der Druckverlust der Zweiphasenströmung $P_{g1}$ ergibt sich aus dem experimentell bestimmten oder wie oben erläutert abgeschätzten Druckverlust der reinen strömenden Flüssigphase $P_1$ dann mit

$$P_{g1} = \phi^2 * P_l$$

Im vorliegenden Fall der Herstellung von Aldehyden durch Hydroformylierung von Olefinen gestaltet sich die Berechung des Druckverlustes noch komplexer. Es ist neben der Synthesegas- und einer flüssigen Katalysatorphase die Gegenwart einer organischen Flüssigphase zu berücksichtigen. Dieser Problematik kann durch die Bestimmung eines weiteren dimensionslosen Druckes $\phi^2_{arg} = P_{g11}/P_{1g}$ Rechnung getragen werden, so daß der Druckverlust wie folgt zu bestimmen ist:

$$P_{gll} = \phi^2 * \phi^2_{org} * P_l$$

Allgemein gilt mit der Reaktorlänge L [m]

$$PD = P_{gl}/L. \quad \text{bzw. } PD = P_{gll}/L$$

Der Druckverlust einer Mehrphasenströmung ist somit durch übliche Mittel der chemischen Verfahrenstechnik berechenbar. Analoges gilt für den zuvor definierten dimensionslosen Druckverlust B, d. h. den Belastungsfaktor des Mehrphasenreaktors.

Die Größe des dimensionslosen Belastungsfaktors B stellt eine notwendige Grundbedingung des erfindungsgemäßen Verfahrens dar; B sollte größer oder gleich 0,8, bevorzugt größer oder gleich 0,9 oder besonders bevorzugt größer oder gleich 1 sein.

Im Bereich B größer oder gleich 0,8 beginnt ein von oben nach unten betriebener Reaktor zu fluten. Es sei ausdrücklich darauf hingewiesen, daß bei Einhaltung dieser Bedingungen die Vorteile des erfindungsgemäßen Verfahrens auch dann erzielt werden, wenn der Reaktor von unten nach oben oder in anderer Richtung betrieben wird.

Höhere Querschnittsbelastungen des Reaktors (B >> 1), erkennbar am steigenden Differenzdruck über den Reaktor, sind jederzeit möglich und sogar erwünscht, solange die steigenden Raum-Zeit-Ausbeuten den gleichermaßen steigenden Energieverbrauch rechtfertigen. Eine Obergrenze ist daher nur durch praktische Überlegungen wie Energieverbrauch oder Schwierigkeiten bei der Trennung der Phasen nach erfolgter Reaktion gegeben.

Es ist somit ersichtlich, daß neben den Volumenströmen der einzelnen Phasen bzw. den hieraus abgeleiteten Leerrohrgeschwindigkeiten $w = V/(\pi D^2/4)$ die Apparateabmessungen des Reaktors (Länge L, Durchmesser D) sowie insbesondere die Daten der verwendeten Füllkörper (hydraulischer Durchmesser $d_H$, Leerrohranteil $\Psi$) eine wichtige Rolle spielen. Über die richtige Wahl dieser Parameter kann das Verfahren unschwer an die unterschiedlichsten Erfordernisse angepaßt werden, wichtig ist nur die Einhaltung der Forderung B >= 0,8, bevorzugt B >= 0,9 und besonders bevorzugt B >= 1.

Bei einer langsamen Reaktion wird man beispielsweise den hydraulischen Durchmesser der Füllkörper klein bzw. ihre spezifische Oberfläche groß wählen, so daß die geforderten Bedingungen für B schon bei kleinen Strömungsgeschwindigkeiten erreicht werden. Man erhält auf diese Weise ausreichende Verweilzeiten über die Länge eines technisch vernünftig dimensionierten Reaktors. Bei sehr schnellen Reaktionen empfiehlt sich eine umgekehrte Verfahrensweise.

Ein weiteres Kriterium bei der Durchführung des erfindungsgemäßen Verfahrens ist das Verhältnis des Massenstroms der flüssigen, den Katalysator enthaltenden Phase $M_1$ zu dem Massenstrom der oder den dispersen Phasen $M_2$. Im vorliegenden Fall der Hydroformylierung ist der Massenstrom der Katalysatorphase $M_1$ wesentlich größer als der Massenstrom der dispersen Phasen, d. h. der organischen Olefinphase $M_{2a}$ und der Synthesegasphase $M_{2b}$ Im erfindungsgemäßen Verfahren kann das Massenverhältnis $M_1/M_2$ der kontinuierlichen Phase ($M_1$) zu den dispersen Phasen ($M_2$) größer 2 sein, vorzugsweise gilt $M_1/M_2 > 10$. Strömungsverhältnisse mit $M_1/M_2 > 100$ sind durchaus möglich und häufig sogar vorteilhaft. Unter der Bedingung $M_1/M_2 > 2$ ist die Katalysatorphase die kontinuierliche Phase, während die dispersen Phasen in feine Blasen bzw. in feine Tropfen zerteilt werden. Im erfindungsgemäßen Verfahren ist es möglich, daß mindestens ein Edukt (Olefin) durch die von der kontinuierlichen Phase (Katalysator) in den Rohrreaktor eingebrachte Energie dispergiert wird: Dies führt zu einer Verteilung zumindest eines Edukts in Blasen oder Tropfen innerhalb der kontinuierlichen Katalysatorphase.

Auch dies läßt sich mit üblichen ingenieurtechnischen Mitteln abschätzen. Es eignen sich hierfür Ansätze mit dimensionslosen Kennzahlen wie

$$d_S/d_H = k * Re_{gl(gll)}{}^m * We_{gl(gll)}{}^n$$

mit

| | |
|---|---|
| $d_s$ | Durchmesser der Tropfen bzw Blasen nach Sauter (in Brauer et al.) |
| $d_H$ | hydraulischer Füllkörperdurchmesser, |
| $Re_{g1(g11)}$ | Reynoldszahl der Mehrphasenströmung $= w_{g1(g11)} * (\rho_1/\eta_1) * (d_H/\Psi)$, |

| $We_{g1(g11)}$ | Weberzahl der Mehrphasenströmung = $W_{g1(g11)}2 * (\rho_1/\sigma_{g1})* (d_H/\Psi)^2$, |
|---|---|
| k, m, n | empirische Konstanten (bekannt oder durch Versuche zu ermitteln), |
| w | Leerrohrgeschwindigkeiten [m/s] = $V/(\pi D^2/4)$, |
| V | Volumenstrom unter Betriebsbedingungen [m³/s], |
| $\rho$ | Dichte unter Betriebsbedingungen [kg/m3], |
| $\eta$ | Viskosität unter Betriebsbedingungen [Pa*s] und |
| $\gamma$ | Grenzflächenspannung unter Betriebsbedingungen [N/m] |

und den Indices 1 (Flüssigphase), g (Gasphase), gl (Gas/Flüssig-Zweiphasenströmung) und gll (Gas/Flüssig/Flüssig-Dreiphasenströmung).

Bei strukturierten Packungen wie Sulzer-SMV oder engen Rohren als Einbauten scheint plausibel, daß ein berechneter Blasen- bzw. Tropfendurchmesser $d_s$ größer als der Kanaldurchmesser nicht sinnvoll ist. Dies gilt aber nicht für durchlässige Packungen und Füllkörper wie beispielsweise Maschendrahtringe oder Maschendrahtgewebe (sogenannte Demisterpackungen oder Tropfenabscheider). Im erfindungsgemäßen Verfahren können berechnete Tropfendurchmesser verwendet werden, die mindestens gleich oder kleiner als der hydraulische Kanaldurchmesser sind:

$$d_S/d_H <= 1, \text{ vorzugsweise} < 0{,}9.$$

Aus dem berechneten Tropfendurchmesser läßt sich schließlich eine Stoffübergangsfläche nach

$$A_S = 6\varphi_g d_S \ [\text{m}^2/\text{m}^3]$$

berechnen.

Für den Phasenanteil $\varphi_g$ der dispersen Phase (im Falle der Hydroformylierung sind Synthesegas und/oder organische Phase dispergiert), kann mit den Leerrohrgeschwindigkeiten der Phasen

$$\varphi_g \sim w_g/w_{gl}$$

gesetzt werden.

Die Verweilzeit $\tau$ der den Reaktor durchströmenden Phasen läßt sich angenähert nach $\tau \sim L^*\psi/w_{1g}$ berechnen. Die Verweilzeit $\tau$ beträgt bei dem erfindungsgemäßen Verfahren in der Regel weit unter einer Stunde und kann im Minutenbereich oder sogar noch darunter liegen. Dennoch werden bei dieser völlig ungewöhnlichen Fahrweise - hoher Katalysatordurchsatz im Reaktor, vergleichsweise geringer Anteil an Edukt an der Reaktionsmasse, dadurch bedingt wiederum sehr kurze Verweilzeit - überraschend hohe Raum-Zeit-Ausbeuten erzielt werden. Alternativ kann bei gleichen Raum-Zeit-Ausbeuten bei deutlich tieferen Temperaturen als üblich gearbeitet werden, da die Steigerung der Reaktionsgeschwindigkeit, was zum Beispiel die Minimierung von Folgereaktionen und damit verbesserte Selektivität nach sich ziehen kann, dies wirtschaftlich zuläßt.

Das erfindungsgemäße Verfahren kann sehr flexibel den unterschiedlichsten Anforderungen angepaßt werden. Für spezielle Anforderungen bieten sich folgende Ausführungsformen des erfindungsgemäßen Verfahrens an:

Erfordert der Einsatzzweck eine sehr lange Durchmischungszone oder sind Ruhezonen z. B. zur Abnahme von Stoffströmen erforderlich, so bietet sich eine kaskadierende Anordnung von Rohrreaktoren mit Einbauten oder Füllkörpern an.

Eine Kaskadierung von Rohrreaktoren oder die alternative Anordnung von gepackten und leeren Rohrabschnitten ist zu empfehlen, wenn ein besonders geringer Druckverlust gewünscht wird.

Weiterhin ist die parallele Anordnung von Rohrreaktoren oder die Verwendung eines Vielrohrreaktors, wobei die Rohre die Funktion der Einbauten übernehmen können, einsetzbar. Außerdem können Reaktoren mit einer Mehrfacheinspeisung von Gas über die Reaktorlänge versehen werden, wenn der Gasverbrauch so hoch ist, daß ungünstige Phasenverhältnisse von Gas zu Flüssigkeit bei der Zusammenführung der beiden Phasen vor dem Reaktor resultieren.

Die besonderen Bedingungen des erfindungsgemäßen Verfahrens lassen weitere Ausführungsformen des Verfahrens zu. So kann der hohe notwendige Kreislauf der Katalysatorphase bzw. der kontinuierlichen Phase zusätzlich zum Betrieb einer Strahldüse, die als Flüssigkeitsstrahl-Gasverdichter vor dem eigentlichen Rohrreaktor angeordnet wird, ausgenutzt werden. Dies kann zur gründlichen Vorvermischung der beiden Phasen sowie zur Verdichtung der Gasphase, was die Fahrweise bei höheren Vordrücken im Reaktor ermöglicht, eingesetzt werden. Dies bietet sich beim Einsatz von gasförmigen Olefinen an. Schließlich wird, wenn man umgekehrt statt der Verdichtung des Gases die Saugwirkung ausnutzt, sogar eine Kreislaufführung von Gas unter gleichzeitiger Vorvermischung der Phasen möglich. Die von der den Katalysator enthaltende kontinuierliche Phase in den Rohrreaktor eingebrachte Energie kann so zur Dispergierung der Eduktphase bzw. mindestens eines Edukts eingesetzt werden.

Auch die Wärmeabfuhr bei stark exothermen Reaktionen wie der Hydroformylierung von Olefinen ist beim erfindungsgemäßen Verfahren unkritisch. Der hohe Durchsatz des Katalysatorkreislaufs wirkt als Wärmeträger, so daß selbst bei adiabatischer Fahrweise des Reaktors nur geringe Temperaturdifferenzen auftreten und eine homogene Temperaturverteilung im Reaktor ohne Temperaturspitzen resultiert. Die erzeugte Wärme läßt sich dann bequem durch einen beliebig im äußeren Katalysatorkreislauf angeordneten, konventionellen Wärmetauscher abführen oder zur Energiegewinnung ausnutzen. Zur besseren Wärmeabfuhr kann es unter Umständen günstig sein, den Katalysatorkreislauf noch höher zu fahren (also bei einem höheren B-Wert) als technisch notwendig ist, da über den Katalysatorkreislauf ein kleiner Temperaturgradient über den Reaktor einstellbar ist.

Das erfindungsgemäße Verfahren bietet im Vergleich zum Stand der Technik erhebliche Vorteile, genannt seien:

- Bei vergleichsweise niedrigen Temperaturen können hohe Raum-Zeit-Ausbeuten erreicht werden.
- Die Bildung von Nebenprodukten ist extrem niedrig, Werte von 1 - 2 Gew.-% und darunter sind möglich.
- Der Katalysator wird geschont, die Desaktivierung ist sehr gering, eine kontinuierliche Ausschleusung entfällt.

Im vorliegenden Falle der Herstellung von Aldehyden durch Hydroformylierung von Olefinen mit dem erfindungsgemäßen Verfahren kommen weitere Vorteile hinzu:

- Aufgrund der höheren Reaktionsgeschwindigkeit kann dieses Verfahren auch für die Hydroformylierung von höheren Olefinen mit mehr als 6 Kohlenstoffatomen wirtschaftlich genutzt werden.
- Bei gasförmigen Olefinen kann der nach einem Teilumsatz verbleibende Eduktanteil durch einfache Rückführung mittels einer Strahldüse zurückgeführt werden.

Im erfindungsgemäßen Verfahren ist die Katalysatorphase die kontinuierliche Phase; ein Massenverhältnis zwischen der Katalysatorphase und der oder den dispersen Phasen, d. h. der oder den olefinischen Phase(n) am Reaktoreingang im Bereich 5000/1 bis 4/1, vorzugsweise im Bereich 2000/1 bis 50/1 ist zweckmäßig. Das Massenverhältnis zwischen Katalysatorphase und dem Hydroformylierungsagens (in der Regel Synthesegas) beträgt 4/1 bis 10000/1, vorzugsweise 200/1 bis 4000/1.

Die Reaktanten können vorgewärmt, d. h. im Bereich der Reaktionstemperatur, oder kalt zugeführt werden. Aufgrund des hohen Phasenverhältnisses mit der Katalysatorphase kann die Vorwärmung auch durch die Prozeßwärme erfolgen.

Das erfindungsgemäße Verfahren zur Hydroformylierung von Olefinen wird bevorzugt in einem Temperaturbereich von 20 °C bis 250 °C, besonders bevorzugt im Bereich von 90 °C bis 150 °C durchgeführt. Hierbei liegt der Gesamtdruck zwischen 10 bar und 300 bar, vorzugsweise zwischen 20 bar und 150 bar.

Der Rohrreaktor kann im Gleichstrom von oben nach unten oder umgekehrt durchströmt werden. Aus Sicherheitsgründen wird der Beschickung von oben der Vorzug gegeben.

Die Reaktionswärme kann über verschiedene Wärmeaustauscher abgeführt werden. Die Wärmeaustauscher müssen hierbei nicht in der Nähe des Reaktionsraums sein, sondern können auch beliebig außerhalb des Reaktors liegen. Die einzelnen Wärmeflüsse sind abhängig von der spezifischen Reaktionswärme sowie von den gewünschten Temperaturen im Reaktor und in den Aufarbeitungsvorrichtungen.

Die abgeführte Reaktionswärme kann so sehr einfach genutzt werden, z.B. im Prozess selbst, zur Beheizung einer Destillationseinrichtung oder zur Erzeugung von Dampf.

Das den Reaktor verlassende Gemisch kann für den Fall des Einsatzes von gasförmigen Olefinen oder bei einem unvollständigen Umsatz in einem Gas-Flüssig-Trennbehälter entgast werden. Die Gas-Flüssigkeit-Trennung kann beim gleichen Druck, der am Reaktorausgang herrscht, erfolgen. Dies ist besonders dann von Vorteil, wenn zumindestens ein Teil des Entspannungsgas in den Reaktor zurückgeführt wird. Ansonsten kann auch bei tieferem Druck (bis hinunter auf 1 bar) entspannt werden.

Der abgetrennte Gasstrom kann vollständig oder teilweise in den Reaktor zurückgeführt werden.

Diese Rückführung kann auf bekannte Weise, z.B. durch eine Strahl- oder Mischdüse, die im Katalysatorkreislaufstrom vor dem Reaktor angebracht ist, oder durch einen Kreisgasverdichter, erreicht werden. Aus energetischen Überlegungen wird bevorzugt eine Strahl- oder Mischdüse, die im Katalysatorkreislaufstrom vor dem Reaktor angebracht ist, verwendet.

Die restliche oder wahlweise die gesamte Gasmenge kann gekühlt oder ungekühlt in ein Abgasverwertungssystem eingeleitet werden. Bei Verwendung eines Kühlers kann das im Kühler anfallende Gaskondensat über eine Leitung in den Gas-Flüssig-Trennbehälter geleitet werden.

Das entgaste Flüssigkeitsgemisch wird in einem Flüssig-Flüssig-Trennbehälter in die Katalysatorphase und in die Produktphase mechanisch getrennt. Dies kann in Absitzbehältern verschiedener Bauart oder Zentrifugen erfolgen. Aus Kostengründen werden Absitzbehälter bevorzugt.

Die Verweilzeiten in der Trennvorrichtung sind zwar nicht grundsätzlich kritisch, werden aber vorteilhaft klein gehalten. Dies hat folgende Vorteile: Die Trennvorrichtung ist klein und das Investment dafür entsprechend gering. Bei kurzen Verweilzeiten treten praktisch keine Nebenreaktionen im Trennbehälter auf. Damit die Trennung der Phasen schnell erfolgt, muß der Dichteunterschied der beiden Phasen genügend groß und deren Viskositäten gering sein. Alle drei Größen sind eine Funktion der Temperatur und können durch orientierende Versuche leicht ermittelt werden.

Darüber hinaus kann die Dichte und Viskosität der Katalysatorlösung durch Auswahl des Solvens und der Katalysatorkonzentration variiert werden. Als weitere Möglichkeit kann die Dichte und Viskosität der Produktphase durch Zusatz eines Lösungsmittel verändert werden.

Die Phasentrennung kann in einem weiten Temperaturbereich vorgenommen werden. Dabei kann die Trenntemperatur auch höher sein als die Temperatur des Reaktionsaustrags am Reaktorausgang. Aus energetischen Gründen ist es jedoch nicht günstig, eine höhere Temperatur als die Flüssigkeitstemperatur im Gasabscheider anzuwenden. Als tiefstmögliche Temperatur ist der Stockpunkt einer der beiden flüssigen Phasen anzusehen. Im Hinblick auf kurze Trennzeiten werden jedoch, wie oben erwähnt, keine zu tiefen Temperaturen gewählt.

Der Produktstrom kann nach bekannten Verfahren aufgetrennt werden, z.B. durch Destillation.

Die abgetrennte Katalysatorlösung wird, ggf. nach Ausschleusung einer kleinen Teilmenge und entsprechendem Ersatz durch frische Katalysatorlösung, in den Reaktor zurückgeführt.

Die folgenden Beispiele sollen die Erfindung beschreiben, ohne deren Anwendungsbreite einzuschränken, die aus den Patentansprüchen hervorgeht.

**Hydroformylierung von Propen:**

**Beispiel 1 (Vergleichsbeispiel, Batchreaktion)**

In einem Rührautoklaven wurden 290,3 g des TPPTS-Liganden (Triphenylphosphintrisulfonat) in Form seines Natriumsalzes, 31,8 g Propen und ein Teil von 291 g eines Lösungsmittels bestehend aus 20 Gew.-% Ethylenglykol und 80 Gew.-% Wasser, bei 120 °C und 50 bar Synthesegas vorgelegt. Die Hydroformylierungsreaktion wurde durch Zugabe von 0,531 g Rhodiumacetat gelöst im Rest des Lösungsmittels gestartet. Nach vollständigem Umsatz, der über die Synthesegasaufnahmekurve detektiert werden konnte, wurde dem Reaktionsgemisch eine flüssige Probe entnommen.

| Beispiel | 1 |
|---|---|
| Substanz | Anteil Gew.-% |
| Propen | 0,64 |
| i-Butyraldehyd | 5,90 |
| n-Butyraldehyd | 75,76 |
| n-Butanol | 1,69 |
| i-Propyl 1,3-Dioxolan | 0,74 |
| n-Propyl 1,3-Dioxolan | 7,33 |
| Schwersieder | 7,94 |

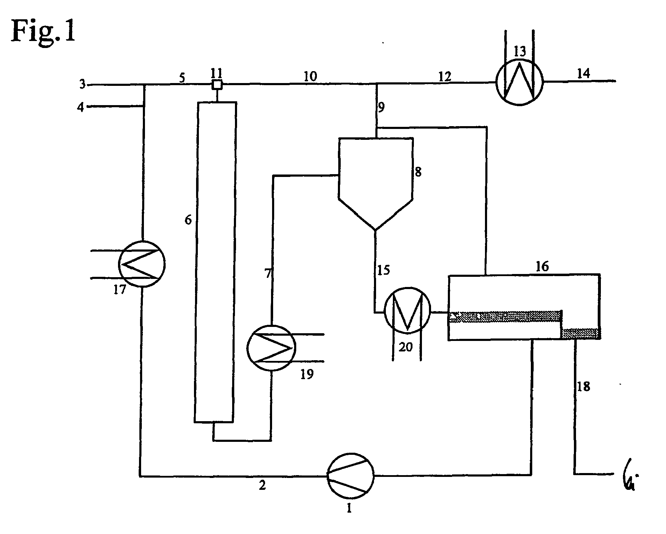

Alle kontinuierlichen Hydroformylierungsversuche (auch jene mit einem anderen Edukt als Propen) erfolgten in einer Versuchsapparatur, die schematisch in Fig. 1 dargestellt ist. Es wurde, wenn bei der Beispielsbeschreibung nicht ein anderer Reaktor genannt wurde, ein Reaktor mit einer Länge von 3 m und einem Durchmesser von 17,3 mm (Volumen 705 ml) eingesetzt, der statische Mischelemente der Fa. Sulzer mit einem hydraulischen Durchmesser von 2 mm enthielt. Der wässrige Katalysators wird mit einer Pumpe 1 im Kreislauf gepumpt. Zur Katalysatorlösung wird Olefin (Propen) 3 und Synthesegas 4 zugemischt. Die so erhaltene Mehrphasenmischung 5 wird über die Mischdüse 11 durch den Rohrreaktor 6 gepumpt, der mit statischen Mischelementen versehen ist. An dieser Stelle ist die innige Vermischung der Phasen, die bei gegebenen Mischelementen eine Funktion der Reynoldszahl sind, von besonderer Bedeutung. Die resultierende Mischung 7, bestehend aus Produkt, nicht umgesetztem Edukt und dem Katalysator werden im Behälter 8 entgast. Das Gas 9, bestehend aus Olefin (Propen), Synthesegas und angereicherten Inerten wird zum größten Teil dem Reaktor 6 über eine Gasrückführungsleitung 10 mit Hilfe einer Mischdüse 11 erneut zugeführt. Einer kleiner Teil des Gasstroms 9 wird über Leitung 12 ausgeschleust. Durch geeignete Kühlung 13 und Rücklauf des überkritischen Propens reduziert sich die Ausschleusung 14 auf angereicherte Inerte und kleine Mengen nicht umgesetzten Synthesegases.

Durch diese Anordnung wird der Umsatz von Olefin (Propen) praktisch nicht durch die Ausschleusung von Inerten limitiert.

Der nach der Entgasung im Behälter 8 anfallende Flüssigkeitsstrom 15 wird in einen Phasentrennbehälter 16 geleitet. Hier wird die wäßrige Katalysatorphase 2 abgetrennt und erneut dem Kreislauf zugeführt. Die Reaktionswärme kann elegant über einen außenliegenden Wärmetauscher 17 abgeführt werden.

**Beispiele 2 bis 5 (Vergleichsbeispiele, kontinuierlicher Prozeß ohne Lösungsmittelgemisch)**

Diese Beispiele stellen Vergleichsversuche in der beschriebenen kontinuierlichen Apparatur dar, um die Vorteile der vorliegenden Erfindung in Bezug auf die Raum-Zeit-Ausbeute gegenüber einem rein wäßrigen Lösungsmittel herauszustellen. Für diese Beispiele war die Leitung 10 zur Rückführung des Gases geschlossen. Als Lösungsmittel für den Katalysator wurde Wasser eingesetzt. Der Reaktor mit einem Volumen von 705 ml wurde mit einer Katalysatorbelastung von 400 kg/h bei einer Temperatur von 120°C durchströmt. Der Reaktionsdruck betrug 50 bar. Die Konzentration des Rhodiums betrug 800 ppm bezogen auf die Lösungsmittelphase. Als Ligand wurde TSTPP in Form seines Natriumsalzes (NaTSTPP) eingesetzt, das Verhältnis P/Rh betrug 60. Für das Beispiel 3 wurden die Reaktionsbedingungen von Beispiel 2 eingestellt, mit der Abweichung, dass die Reaktionstemperatur 130°C betrug. Für den Versuch 4 wurden die Reaktionsbedingungen von Beispiel 2 eingestellt, mit der Abweichung, dass der Reaktionsdruck 70 bar betrug. Für das Beispiel 5 wurden die Reaktionsbedingungen von Beispiel 2 eingestellt, mit der Abweichung, dass die Katalysatorbelastung des Reaktors 300 kg/h betrug. Die zugefahrenen Stoffmengenströme der Edukte sowie der Produkte sind in der Tabelle in mol/h angegeben.

| Beispiel | 2 | 3 | 4 | 5 |
|---|---|---|---|---|
| RZA [t/(m$^3$*h)] | 0,98 | 1,39 | 1,23 | 0,71 |
| Edukt | | | | |
| CO | 22,79 | 16,99 | 28,24 | 12,73 |
| H$_2$ | 21,28 | 15,87 | 26,38 | 11,89 |
| N$_2$ | 0,12 | 0,09 | 0,15 | 0,07 |
| Propen | 51,49 | 35,57 | 31,13 | 33,45 |
| Propan | 0,17 | 0,12 | 0,10 | 0,11 |
| Produkt | | | | |
| CO | 2,15 | 2,09 | 2,08 | 2,11 |
| H$_2$ | 0,89 | 0,93 | 0,98 | 0,97 |
| Propen | 3,35 | 6,73 | 3,04 | 4,00 |
| i-Butanal | 0,24 | 0,51 | 0,53 | 0,20 |
| n-Butanal | 5,46 | 11,03 | 9,82 | 4,66 |
| i-Butanol | <0,01 | <0,01 | <0,01 | <0,01 |
| n-Butanol | 0,07 | 0,07 | 0,07 | 0,07 |
| 2-Ethylhexanal | 0,09 | 0,03 | 0,08 | 0,02 |
| 2-Ethylhexenal | 0,26 | 0,19 | 0,18 | 0,09 |

(fortgesetzt)

| Abgas | | | | |
|---|---|---|---|---|
| CO | 9,50 | 3,06 | 13,23 | 6,81 |
| $H_2$ | 8,45 | 1,46 | 9,97 | 3,59 |
| $N_2$ | 0,29 | 0,10 | 0,09 | 0,16 |
| Propen | 37,37 | 13,56 | 17,72 | 36,93 |
| Propan | 0,17 | 0,10 | 0,17 | 0,09 |
| i-Butanal | 0,08 | 0,08 | 0,03 | 0,01 |
| n-Butanal | 4,59 | 3,13 | 2,72 | 2,56 |

**Beispiele 6 bis 11 (gemäß der Erfindung)**

Diese Beispiele beschreiben den erfindungsgemäßen Einsatz von Lösungsmittelgemischen in der beschriebenen kontinuierlichen Apparatur am Beispiel von Wasser/Ethylenglykol. Die gemessenen Daten verdeutlichen die gesteigerten Raum-Zeit-Ausbeuten. Hierbei wurde keine nennenswerte Bildung von Dioxolanen im Gegensatz zu gerührten Systemen verzeichnet. Im Beispiel 6 wurden die Reaktionsbedingungen von Beispiel 5 eingestellt, mit der Abweichung, dass als Lösungsmittel ein Gemisch aus Wasser und Ethylenglykol (20 Gew.-%) verwendet wurde; im Beispiel 7 wurde die Konzentration von Ethylenglykol auf 40 Gew.-% gesteigert. Im Beispiel 8 wurden die Reaktionsbedingungen von Beispiel 7 eingestellt, mit der Abweichung, dass die Katalysatorbelastung des Reaktors 400 kg/h und die Reaktionstemperatur 130°C betrug. Im Beispiel 9 wurden die Reaktionsbedingungen von Beispiel 8 eingestellt, mit der Abweichung, daß die Reaktionstemperatur 120°C und der Reaktionsdruck 70 bar betrug. Im Beispiel 10 wurden die Reaktionsbedingungen von Beispiel 9 eingestellt, mit der Abweichung, dass die Reaktionstemperatur 90°C betrug. Dieser Versuch dokumentiert die Möglichkeit, die Hydroformylierung von Propen im Mehrphasensystem auch bei tieferen Temperaturen noch mit hohen Raum-Zeit-Ausbeuten durchzuführen. Im Beispiel 11 wurden die Reaktionsbedingungen von Versuch 10 eingestellt, mit der Abweichung, daß der Reaktionsdruck 50 bar und die Rhodiumkonzentration 200 ppm bezogen auf das Lösungsmittel betrug. Dieser Versuch dokumentiert die Möglichkeit die Hydroformylierung von Propen im Mehrphasensystem auch mit niedrigen Rhodiumkonzentrationen noch mit hohen Raum-Zeit-Ausbeuten durchzuführen. Die zugefahrenen Stoffmengenströme der Edukte sowie der Produkte sind in der Tabelle in mol/h angegeben.

| **Beispiel** | **6** | **7** | **8** | **9** | **10** | **11** |
|---|---|---|---|---|---|---|
| RZA [t/m$^3$/h] | 1,3 | 1,7 | 3,8 | 2,3 | 0,3 | 0,5 |
| Edukt | | | | | | |
| CO | 23,31 | 23,28 | 53,28 | 45,72 | 22,96 | 23,23 |
| $H_2$ | 21,40 | 21,57 | 49,16 | 42,38 | 21,70 | 21,33 |
| $N_2$ | 0,12 | 0,11 | 0,25 | 0,20 | 0,12 | 0,09 |
| Propen | 49,88 | 35,20 | 59,35 | 42,89 | 38,04 | 35,59 |
| Propan | 0,12 | 0,08 | 0,12 | 0,09 | 0,08 | 0,07 |
| Produkt | | | | | | |
| CO | 2,25 | 8,55 | 2,05 | 2,50 | 0,61 | 0,29 |
| $H_2$ | 0,83 | 1,51 | 0,41 | 1,02 | 0,16 | 0,10 |
| Propen | 4,37 | 5,38 | 6,33 | 4,14 | 0,51 | 0,18 |
| i-Butanal | 0,55 | 1,20 | 3,47 | 2,13 | 0,06 | 0,03 |
| n-Butanal | 9,34 | 17,25 | 36,99 | 21,60 | 0,96 | 0,52 |
| i-Butanol | 0,00 | 0,00 | 0,03 | 0,02 | 0,00 | 0,00 |

(fortgesetzt)

| Produkt | | | | | | |
|---|---|---|---|---|---|---|
| n-Butanol | 0,06 | 0,15 | 0,48 | 0,23 | 0,01 | 0,00 |
| i-Propyldioxolan | <0,01 | 0,02 | 0,04 | 0,03 | <0,01 | <0,01 |
| n-Propyldioxolan | 0,05 | 0,21 | 0,46 | 0,37 | 0,13 | 0,12 |
| 2-Ethylhexanal | 0,20 | 0,21 | 0,46 | 0,39 | 0,08 | 0,05 |
| 2-Ethylhexenal | 0,41 | 0,50 | 0,60 | 0,58 | 0,10 | 0,17 |
| Abgas | | | | | | |
| CO | 9,09 | 3,15 | 5,79 | 16,42 | 18,42 | 18,37 |
| $H_2$ | 5,05 | 1,20 | 1,85 | 11,61 | 14,90 | 14,29 |
| $N_2$ | 0,22 | 0,09 | 0,33 | 0,25 | 0,13 | 0,12 |
| Propen | 30,09 | 6,31 | 9,21 | 13,04 | 35,28 | 34,04 |
| Propan | 0,18 | 0,06 | 0,17 | 0,20 | 0,10 | 0,10 |
| i-Butanal | 0,09 | 0,04 | 0,01 | 0,26 | 0,13 | 0,20 |
| n-Butanal | 3,43 | 0,36 | 1,35 | 1,87 | 1,61 | 2,95 |

## Selektivhydroformylierung von 1-Buten

Eine weitere Anwendung des erfindungsgemäßen Verfahrens ist die selektive Hydroformylierung von 1-Buten aus einem Raffinat I-Gemisch. Dieses Gemisch bestehend aus aus $C_4$-Olefinen und $C_4$-Paraffinen enthält Anteile an 1-Buten von 26-29 Gew.-%. Als Katalysatorlösungsmittel wurde eine Mischung aus Wasser und Ethylenglykol (50/50 Gew.-%) gewählt.

## Beispiel 12 (Vergleichsbeispiel, Batch-Prozeß)

Dieses Beispiel stellt einen Vergleichsversuch im Rührautoklaven dar, um die Vorteile der vorliegenden Erfindung in Bezug auf die Produktqualität gegenüber herkömmlichen Rührreaktoren herauszustellen. In einem Rührautoklaven wurden 29,81 g NaTSTPP, 86,2 g 1-Buten, 13,5 g Isobutan und 67,7 g eines Lösungsmittels, bestehend aus 30 Gew.-% Ethylenglykol und 70 Gew.-% Wasser, bei 105 °C und 30 bar Synthesegas vorgelegt. Die Hydroformylierungsreaktion wurde durch Zugabe von 2,09 g einer 3,7 % Rh enthaltenen Rh-Acetat-Lösung (30 Gew.-% Ethylenglycol und 70 Gew.-% Wasser) gestartet. Nach vollständigem Umsatz, der über die Synthesegasaufnahmekurve detektiert werden konnte, wurde dem Reaktionsgemisch eine flüssige Probe entnommen. Für die folgende Auflistung wurde Isobutan, das als innerer Standard diente, herausgerechnet.

| Beispiel | 12 |
|---|---|
| **Substanz** | **Anteil Gew.-%** |
| 1-Buten | 6,1 |
| 2-Methylbutanal | 1,19 |
| Pentanal | 77,4 |
| i-Propyl 1,3-Dioxolan | 0,13 |
| n-Propyl 1,3-Dioxolan | 11,9 |
| Rest | 3,28 |

Die kontinuierliche Hydroformylierung von 1-Buten in den Beispielen 13 bis 26 wurde, abgesehen davon, dass die Gasrückführleitung 10 geschlossen war, wie die Propen-Hydroformylierung durchgeführt.

**Beispiele 13:**

Dieses Beispiel beschreibt den erfindungsgemäßen Einsatz eines Lösungsmittelgemisches aus 50 Gew.-% Wasser und 50 Gew.-% Ethylenglykol in der beschriebenen kontinuierlichen Apparatur. Aus den gemessenen Daten wird die deutlich verringerte Bildung von Dioxolanen im Gegensatz zu gerührten Systemen dokumentiert. Der Reaktor von 3 m Länge wurde mit einer Katalysatorbelastung von 400 kg/h bei einer Temperatur von 115°C durchströmt. Hierzu wurden 600 Nl/h Synthesegas und 3 kg/h Raffinat I zugefahren. Der Reaktionsdruck betrug 50 bar. Folgende Daten wurden ermittelt:

| Beispiel | 13 |
|---|---|
| 1-Buten, Edukt Gew.-% | 27,00 |
| U (1-Buten) | 64,68% |
| RZA [t/m$^3$/h] | 0,74 |
| Produkt: | |
| i-Butan | 1,39 |
| n-Butan | 5,77 |
| Cyclobutan | 0,04 |
| trans-Buten | 5,93 |
| 1-Buten | 5,07 |
| iso-Buten | 21,77 |
| cis-Buten | 4,14 |
| Neo-Pentan | 0,04 |
| 1,3-Butadien | 0,00 |
| 2-Methylbutanal | 1,88 |
| 3-Methylbutanal | 5,28 |
| n-Pentanal | 42,58 |
| 2-Methylbutanol | 0,12 |
| Methylpropyl-Dioxolan | 0,09 |
| 1-Pentanol | 0,41 |
| 2-n-Butyl 1,3Dioxolan | 0,25 |
| Ethylenglykol | 0,03 |

**Beispiel 14:**

Es wurden die Reaktionsbedingungen des Beispiels 13 eingestellt, mit der Abweichung, daß die Katalysatorbelastung des Reaktors 200 kg/h betrug.

| Beispiel | 14 |
|---|---|
| 1-Buten, Edukt Gew.-% | 27,30 |
| U (1-Buten) | 59,46 % |
| RZA [t/m$^3$/h] | 0,69 |
| Produkt: | |
| i-Butan | 3,10 |

(fortgesetzt)

| Produkt: | |
|---|---|
| n-Butan | 9,71 |
| Cyclobutan | 0,06 |
| trans-Buten | 8,55 |
| 1-Buten | 9,50 |
| iso-Buten | 35,98 |
| cis-Buten | 5,60 |
| Neo-Pentan | 0,06 |
| 1,3-Butadien | 0,00 |
| 2-Methylbutanal | 1,03 |
| 3-Methylbutanal | 2,25 |
| n-Pentanal | 22,07 |
| 2-Methylbutanol | <0,01 |
| Methylpropyl-Dioxolan | <0,01 |
| 1-Pentanol | 0,22 |
| 2-n-Butyl 1,3Dioxolan | 0,23 |
| Ethylenglykol | <0,01 |

**Beispiel 15:**

Es wurden die Reaktionsbedingungen von Beispiel 13 eingestellt, mit der Abweichung, daß die Katalysatorbelastung des Reaktors 100 kg/h und die Reaktionstemperatur 85 °C betrug.

| Beispiel | 15 |
|---|---|
| 1-Buten, Edukt Gew.-% | 28,90 |
| U (1-Buten) | 17,09 % |
| RZA [t/m³/h] | 0,21 |
| Produkt: | |
| i-Butan | 1,98 |
| n-Butan | 10,50 |
| Cyclobutan | 0,07 |
| trans-Buten | 9,89 |
| 1-Buten | 22,17 |
| iso-Buten | 37,42 |
| cis-Buten | 6,19 |
| Neo-Pentan | 0,09 |
| 1,3-Butadien | 0,11 |
| 2-Methylbutanal | 0,29 |
| 3-Methylbutanal | 0,48 |

(fortgesetzt)

| Produkt: | |
|---|---|
| n-Pentanal | 9,61 |
| 2-Methylbutanol | 0,03 |
| Methylpropyl-Dioxolan | 0,03 |
| 1-Pentanol | 0,23 |
| 2-n-Butyl 1,3Dioxolan | 0,19 |
| Ethylenglykol | <0,01 |

**Beispiel 16:**

Es wurden die Reaktionsbedingungen von Beispiel 13 eingestellt, mit der Abweichung, daß die Katalysatorbelastung des Reaktors 250 kg/h und die Reaktionstemperatur 85 °C betrug.

| Beispiel | 16 |
|---|---|
| 1-Buten, Edukt Gew.-% | 28,90 |
| U (1-Buten) | 20,98 % |
| RZA [t/(m$^3$·h)] | 0,26 |
| Produkt: | |
| i-Butan | 1,88 |
| n-Butan | 8, 53 |
| Cyclobutan | 0,06 |
| trans-Buten | 8,88 |
| 1-Buten | 17,07 |
| iso-Buten | 28,61 |
| cis-Buten | 5,48 |
| Neo-Pentan | 0,09 |
| 1,3-Butadien | 0,00 |
| 2-Methylbutanal | 0,77 |
| 3-Methylbutanal | 0,68 |
| n-Pentanal | 27,55 |
| 2-Methylbutanol | 0,05 |
| Methylpropyl-Dioxolan | 0,01 |
| 1-Pentanol | 0,11 |
| 2-n-Butyl 1,3Dioxolan | 0,04 |
| Ethylenglykol | <0,01 |

**Beispiel 17:**

Es wurden die Reaktionsbedingungen von Beispiel 13 eingestellt, mit der Abweichung, daß die Reaktionstemperatur 90 °C betrug.

| Beispiel | 17 |
|---|---|
| 1-Buten, Edukt Gew.-% | 28,90 |
| U (1-Buten) | 27,11 % |
| RZA [t/(m$^3$*h)] | 0,33 |
| Produkt: | |
| i-Butan | 2,19 |
| n-Butan | 8,97 |
| Cyclobutan | 0,06 |
| trans-Buten | 8,94 |
| 1-Buten | 17,11 |
| iso-Buten | 32,67 |
| cis-Buten | 5,53 |
| Neo-Pentan | 0,07 |
| 1,3-Butadien | 0,00 |
| 2-Methylbutanal | 0,76 |
| 3-Methylbutanal | 0,57 |
| n-Pentanal | 22,99 |
| 2-Methylbutanol | 0,05 |
| Methylpropyl-Dioxolan | 0,00 |
| 1-Pentanol | 0,07 |
| 2-n-Butyl 1,3Dioxolan | 0,03 |
| Ethylenglykol | <0,01 |

**Beispiel 18:**

Es wurden die Reaktionsbedingungen von Beispiel 13 eingestellt, mit der Abweichung, daß die Reaktionstemperatur 70 °C betrug.

| Beispiel | 18 |
|---|---|
| 1-Buten, Edukt Gew.-% | 27,00 |
| U (1-Buten) | 9,39 % |
| RZA [t/(m$^3$*h)] | 0,11 |
| Produkt: | |
| i-Butan | 2,51 |
| n-Butan | 10, 96 |
| Cyclobutan | 0,06 |
| trans-Buten | 9,80 |
| 1-Buten | 23,13 |
| iso-Buten | 39,01 |
| cis-Buten | 6,38 |

(fortgesetzt)

| Produkt: | |
|---|---|
| Neo-Pentan | 0,06 |
| 1,3-Butadien | 0,17 |
| 2-Methylbutanal | 0,24 |
| 3-Methylbutanal | 0,63 |
| n-Pentanal | 4,79 |
| 2-Methylbutanol | 0,02 |
| Methylpropyl-Dioxolan | 0,08 |
| 1-Pentanol | 0,3 |
| 2-n-Butyl 1,3Dioxolan | 0,31 |
| Ethylenglykol | <0,01 |

**Beispiel 19:**

Es wurden die Reaktionsbedingungen von Beispiel 13 eingestellt, mit der Abweichung, daß die Reaktionstemperatur 80 °C betrug.

| Beispiel | 19 |
|---|---|
| 1-Buten, Edukt Gew.-% | 27,00 |
| U (1-Buten) | 19,71 % |
| RZA [t/(m$^3$*h)] | 0,23 |
| Produkt: | |
| i-Butan | 2,01 |
| n-Butan | 10,91 |
| Cyclobutan | 0,08 |
| trans-Buten | 11,71 |
| 1-Buten | 18,23 |
| iso-Buten | 28,57 |
| cis-Buten | 8,01 |
| Neo-Pentan | 0,12 |
| 1,3-Butadien | 0,15 |
| 2-Methylbutanal | 0,67 |
| 3-Methylbutanal | 0,41 |
| n-Pentanal | 17,79 |
| 2-Methylbutanol | 0,04 |
| Methylpropyl-Dioxolan | 0,04 |
| 1-Pentanol | 0,25 |
| 2-n-Butyl 1,3Dioxolan | 0,02 |
| Ethylenglykol | <0,01 |

**Beispiel 20:**

Es wurden die Reaktionsbedingungen von Beispiel 13 eingestellt, mit der Abweichung, daß die Reaktionstemperatur 85 °C betrug.

| Beispiel | 20 |
|---|---|
| 1-Buten, Edukt Gew.-% | 27,00 |
| U (1-Buten) | 24,23 % |
| RZA [t/(m$^3$*h)] | 0,28 |
| Produkt: | |
| i-Butan | 1,68 |
| n-Butan | 9,18 |
| Cyclobutan | 0,06 |
| trans-Buten | 9,01 |
| 1-Buten | 16,27 |
| iso-Buten | 31,89 |
| cis-Buten | 6,34 |
| Neo-Pentan | 0,07 |
| 1,3-Butadien | 0,09 |
| 2-Methylbutanal | 0,78 |
| 3-Methylbutanal | 0,47 |
| n-Pentanal | 19,07 |
| 2-Methylbutanol | 0,06 |
| Methylpropyl-Dioxolan | 0 |
| 1-Pentanol | 0,15 |
| 2-n-Butyl 1,3Dioxolan | 0,03 |
| Ethylenglykol | <0,01 |

**Beispiel 21:**

Es wurden die Reaktionsbedingungen von Beispiel 13 eingestellt, mit der Abweichung, daß die Reaktionstemperatur 95 °C betrug.

| Beispiel | 21 |
|---|---|
| 1-Buten, Edukt Gew.-% | 27,00 |
| U (1-Buten) | 32,66 % |
| RZA [t/(m$^3$*h)] | 0,38 |
| Produkt: | |
| i-Butan | 1,90 |
| n-Butan | 9, 81 |
| Cyclobutan | 0,07 |
| trans-Buten | 10,01 |

(fortgesetzt)

| Produkt: | |
|---|---|
| 1-Buten | 14,99 |
| iso-Buten | 31,62 |
| cis-Buten | 7,04 |
| Neo-Pentan | 0,08 |
| 1,3-Butadien | 0,07 |
| 2-Methylbutanal | 0,82 |
| 3-Methylbutanal | 0,75 |
| n-Pentanal | 22,39 |
| 2-Methylbutanol | 0,05 |
| Methylpropyl-Dioxolan | 0,01 |
| 1-Pentanol | 0,09 |
| 2-n-Butyl 1,3 Dioxolan | 0,02 |
| Ethylenglykol | <0,01 |

**Beispiel 22:**

Es wurden die Reaktionsbedingungen von Beispiel 13 eingestellt, mit der Abweichung, daß die Reaktionstemperatur 105 °C betrug.

| Beispiel | 22 |
|---|---|
| 1-Buten, Edukt Gew.-% | 27,00 |
| U (1-Buten) | 44,34 % |
| RZA [t/(m$^3$*h)] | 0,51 |
| Produkt: | |
| i-Butan | 2,09 |
| n-Butan | 9,47 |
| Cyclobutan | 0,06 |
| trans-Buten | 8,97 |
| 1-Buten | 12, 56 |
| iso-Buten | 34,63 |
| cis-Buten | 6,24 |
| Neo-Pentan | 0,06 |
| 1,3-Butadien | 0,05 |
| 2-Methylbutanal | 0,85 |
| 3-Methylbutanal | 1,08 |
| n-Pentanal | 21,35 |
| 2-Methylbutanol | 0,05 |
| Methylpropyl-Dioxolan | 0,00 |

(fortgesetzt)

| Produkt: | |
|---|---|
| 1-Pentanol | 0,12 |
| 2-n-Butyl 1,3Dioxolan | 0,04 |
| Ethylenglykol | <0,01 |

**Beispiel 23:**

Es wurden die Reaktionsbedingungen von Beispiel 13 eingestellt, mit der Abweichung, daß die Reaktionstemperatur 115 °C betrug.

| Beispiel | 23 |
|---|---|
| 1-Buten, Edukt Gew.-% | 28,10 |
| U (1-Buten) | 66,66 % |
| RZA [t/(m$^3$*h)] | 0,80 |
| Produkt: | |
| i-Butan | 1,71 |
| n-Butan | 7,77 |
| Cyclobutan | 0,05 |
| trans-Buten | 7,37 |
| 1-Buten | 6,19 |
| iso-Buten | 26,46 |
| cis-Buten | 4,87 |
| Neo-Pentan | 0,03 |
| 1,3-Butadien | 0,00 |
| 2-Methylbutanal | 1,8 |
| 3-Methylbutanal | 4,65 |
| n-Pentanal | 36,17 |
| 2-Methylbutanol | 0,09 |
| Methylpropyl-Dioxolan | 0,1 |
| 1-Pentanol | 0,41 |
| 2-n-Butyl 1,3Dioxolan | 0,47 |
| Ethylenglykol | <0,01 |

**Beispiel 24:**

Es wurden die Reaktionsbedingungen von Beispiel 13 eingestellt, mit der Abweichung, daß der Reaktionsdruck 33 bar betrug.

| Beispiel | 24 |
|---|---|
| 1-Buten, Edukt Gew.-% | 28,30 |
| U (1-Buten) | 52,86 % |
| RZA [t/(m$^3$*h)] | 0,64 |

(fortgesetzt)

| Produkt: | |
|---|---|
| i-Butan | 2,63 |
| n-Butan | 10,63 |
| Cyclobutan | 0,06 |
| trans-Buten | 10,22 |
| 1-Buten | 12,20 |
| iso-Buten | 35,78 |
| cis-Buten | 7,45 |
| Neo-Pentan | 0,03 |
| 1,3-Butadien | 0,01 |
| 2-Methylbutanal | 0,48 |
| 3-Methylbutanal | 1,3 |
| n-Pentanal | 17,64 |
| 2-Methylbutanol | 0,04 |
| Methylpropyl-Dioxolan | <0,01 |
| 1-Pentanol | 0,14 |
| 2-n-Butyl 1,3Dioxolan | 0,15 |
| Ethylenglykol | <0,01 |

**Beispiel 25:**

Es wurden die Reaktionsbedingungen von Beispiel 13 eingestellt, mit der Abweichung, daß der Reaktionsdruck 43 bar betrug.

| **Beispiel** | **25** |
|---|---|
| 1-Buten, Edukt Gew.-% | 28,30 |
| U (1-Buten) | 58,59 % |
| RZA [t/(m$^3$*h)] | 0,71 |
| Produkt: | |
| i-Butan | 1,67 |
| n-Butan | 8,02 |
| Cyclobutan | 0,05 |
| trans-Buten | 8,37 |
| 1-Buten | 7,90 |
| iso-Buten | 24,87 |
| cis-Buten | 6,20 |
| Neo-Pentan | 0,04 |
| 1,3-Butadien | 0,01 |
| 2-Methylbutanal | 1,29 |

(fortgesetzt)

| Produkt: | |
|---|---|
| 3-Methylbutanal | 5,18 |
| n-Pentanal | 30,29 |
| 2-Methylbutanol | 0,04 |
| Methylpropyl-Dioxolan | 0,47 |
| 1-Pentanol | 0,79 |
| 2-n-Butyl 1,3Dioxolan | 1,48 |
| Ethylenglykol | <0,01 |

**Beispiel 26:**

Es wurden die Reaktionsbedingungen von Beispiel 13 eingestellt, mit der Abweichung, daß die Reaktorlänge 1 m betrug.

| Beispiel | 26 |
|---|---|
| 1-Buten, Edukt Gew.-% | 27,60 |
| U (1-Buten) | 62,22 % |
| RZA [t/(m$^3$/h)] | 0,73 |
| Produkt: | |
| i-Butan | 2,11 |
| n-Butan | 8,16 |
| Cyclobutan | 0,06 |
| trans-Buten | 8,50 |
| 1-Buten | 7,14 |
| iso-Buten | 25,78 |
| cis-Buten | 5,50 |
| Neo-Pentan | 0,05 |
| 1,3-Butadien | 0,01 |
| 2-Methylbutanal | 1,64 |
| 3-Methylbutanal | 4,57 |
| n-Pentanal | 33,65 |
| 2-Methylbutanol | 0,07 |
| Methylpropyl-Dioxolan | 0,09 |
| 1-Pentanol | 0,41 |
| 2-n-Butyl 1,3Dioxolan | 0,34 |
| Ethylenglykol | <0,01 |

**Beispiel 27:**

Es wurden die Reaktionsbedingungen von Beispiel 13 eingestellt, mit der Abweichung, daß die Reaktorlänge 2 m (bei gleicher Füllung und Durchmesser) betrug.

| Beispiel | 27 |
|---|---|
| 1-Buten, Edukt Gew.-% | 27,60 |
| U (1-Buten) | 62,53 % |
| RZA [t/(m$^3$*h)] | 0,73 |
| Produkt: | |
| i-Butan | 1,38 |
| n-Butan | 6,41 |
| Cyclobutan | 0,04 |
| trans-Buten | 7,16 |
| 1-Buten | 6,02 |
| iso-Buten | 22,28 |
| cis-Buten | 5,35 |
| Neo-Pentan | 0,05 |
| 1,3-Butadien | 0,01 |
| 2-Methylbutanal | 1,34 |
| 3-Methylbutanal | 3,36 |
| n-Pentanal | 36,93 |
| 2-Methylbutanol | 0,04 |
| Methylpropyl-Dioxolan | <0,03 |
| 1-Pentanol | 0,61 |
| 2-n-Butyl 1,3Dioxolan | 0,43 |
| Ethylenglykol | 0,12 |

**Hydroformylierung von 1-Decen**

Der Reaktor der Versuchsapparatur wurde in den Beispielen 28 und 29 mit 400kg/h Katalysatorlösung durchströmt. Die Reaktionstemperatur betrug 125°C und der Reaktionsdruck 70 bar. Die Konzentration des Rhodiums betrug 800 ppm, bezogen auf die Katalysatorphase. Als Ligand wurde TSTPP in Form seines Natriumsalzes eingesetzt.

**Beispiel 28 (Vergleichsbeispiel, kontinuierlicher Prozeß ohne Lösungsmittelgemisch)**

Als Lösungsmittel für den Katalysator wurde Wasser eingesetzt. Der pH-Wert betrug 4,5. Das Verhältnis P/Rh im Katalysator betrug 5. Die zugefahrenen Stoffmengenströme der Edukte sowie der Produkte sind in der Tabelle in mol/h angegeben.

| Beispiel | 28 |
|---|---|
| RZA [t/(m$^3$*h)] | 0,05 |
| B | 13,96 |
| Edukt | |
| CO | 11,38 |
| H$_2$ | 10,52 |
| N$_2$ | 0,05 |

(fortgesetzt)

| Edukt | |
|---|---|
| 1-Decen | 7,12 |
| Produkt | |
| 1-Decen | 6,67 |
| Undecanal | 0,34 |
| 2-Methyldecanal | 0,11 |
| Abgas | |
| CO | 10,36 |
| $H_2$ | 9,89 |
| $N_2$ | 0,05 |

**Beispiel 29 (gemäß der Erfindung)**

Das Beispiel beschreibt den erfindungsgemäßen Einsatz eines Lösungsmittelgemisches aus 50 Gew.-% Wasser und 50 Gew.-% Ethylenglykol für der Hydroformylierung von 1-Decen, um die Überlegenheit der vorliegenden Erfindung in Bezug auf die Raum-Zeit-Ausbeute gegenüber dem Lösungsmittel Wasser zu dokumentieren.

Als Lösungsmittel für den Katalysator wurde ein Gemisch aus Wasser und Ethylenglykol (1:1) eingesetzt. Der pH-Wert betrug 7,3. Das Verhältnis P/Rh im Katalysator betrug 60. Die zugefahrenen Stoffmengenströme der Edukte sowie der Produkte sind in der Tabelle in mol/h angegeben.

| **Beispiel** | **29** |
|---|---|
| RZA [t/(m$^3$*h)] | 0,08 |
| B | 12,94 |
| Edukt | |
| CO | 13,86 |
| $H_2$ | 12,86 |
| $N_2$ | 0,06 |
| 1-Decen | 7,33 |
| Produkt | |
| 1-Decen | 6,65 |
| Undecanal | 0,61 |
| 2-Methyldecanal | 0,04 |
| Abgas | |
| CO | 12,92 |
| $H_2$ | 12,27 |
| $N_2$ | 0,07 |

**Patentansprüche**

1. Verfahren zur Hydroformylierung von einem oder mehreren Olefinen mit je 2 bis 25 Kohlenstoffatomen durch Mehrphasenreaktionen in einem Rohrreaktor
**dadurch gekennzeichnet,**
**daß**

a) der Katalysator in der kontinuierlichen Phase enthalten ist,

b) die kontinuierliche Phase ein Lösungsmittelgemisch enthält, wobei das Lösungsmittelgemisch aus Wasser und einem mit Wasser mischbaren organischen Lösungsmittel, enthaltend mindestens zwei Sauerstoffatome, besteht und eine Dielektrizitätskonstante von 50 bis 78 aufweist.

c) mindestens ein Olefin in der dispersen Phase enthalten ist und

d) der Belastungsfaktor des Rohrreaktors gleich oder größer 0,8 ist.

**2.** Verfahren nach Anspruch 1,
**dadurch gekennzeichnet,**
**daß** der Katalysator ein Metall der 8. Nebengruppe des Periodensystems der Elementen enthält.

**3.** Verfahren nach Anspruch 1 oder 2,
**dadurch gekennzeichnet,**
**daß** der Katalysator Rhodium enthält.

**4.** Verfahren nach einem der Ansprüche 1 bis 3,
**dadurch gekennzeichnet,**
**daß** als Katalysator wasserlösliche Rhodiumverbindungen eingesetzt werden.

**5.** Verfahren nach einem der Ansprüche 1 bis 4,
**dadurch gekennzeichnet,**
**daß** der Belastungsfaktor B größer oder gleich 0,9 ist.

**6.** Verfahren nach einem der Ansprüche 1 bis 5,
**dadurch gekennzeichnet,**
**daß** der Belastungsfaktor B größer oder gleich 1,0 ist.

**7.** Verfahren nach einem der Ansprüche 1 bis 6,
**dadurch gekennzeichnet,**
**daß** das Massenverhältnis der kontinuierlichen Phase zu der oder den dispersen Phasen größer 2 ist.

**8.** Verfahren nach einem der Ansprüche 1 bis 7,
**dadurch gekennzeichnet,**
**daß** die kontinuierliche Phase vor dem Rohrreaktor eine Strahldüse antreibt.

**9.** Verfahren nach einem der Ansprüche 1 bis 8,
**dadurch gekennzeichnet,**
**daß** mindestens ein Edukt durch die von der kontinuierlichen Phase in den Rohrreaktor eingebrachte Energie dispergiert wird.

## Claims

**1.** Process for the hydroformylation of one or more olefins each having from 2 to 25 carbon atoms by means of a multiphase reaction in a tube reactor, **characterized in that**

a) the catalyst is present in the continuous phase,

b) the continuous phase contains a solvent mixture which comprises water and a water-miscible organic solvent containing at least two oxygen atoms and has a dielectric constant of from 50 to 78.

c) at least one olefin is present in the disperse phase and

d) the loading factor of the tube reactor is equal to or greater than 0.8.

**2.** Process according to Claim 1, **characterized in that** the catalyst contains a metal of transition group VIII of the Periodic Table of the Elements.

**3.** Process according to Claim 1 or 2, **characterized in that** the catalyst contains rhodium.

**4.** Process according to any of Claims 1 to 3, **characterized in that** water-soluble rhodium compounds are used as catalyst.

**5.** Process according to any of Claims 1 to 4, **characterized in that** the loading factor B is greater than or equal to 0.9.

**6.** Process according to any of Claims 1 to 5, **characterized in that** the loading factor B is greater than or equal to 1.0.

**7.** Process according to any of Claims 1 to 6, **characterized in that** the mass ratio of the continuous phase to the disperse phase or phases is greater than 2.

**8.** Process according to any of Claims 1 to 7, **characterized in that** the continuous phase drives a jet nozzle upstream of the tube reactor.

**9.** Process according to any of Claims 1 to 8, **characterized in that** at least one starting material is dispersed by means of the energy introduced into the tube reactor by the continuous phase.

### Revendications

**1.** Procédé pour l'hydroformylation d'une ou de plusieurs oléfines avec à chaque fois 2 à 25 atomes de carbone par des réactions en plusieurs phases dans un réacteur tubulaire, **caractérisé en ce que**

a) le catalyseur est contenu dans la phase continue,
b) la phase continue contient un mélange de solvants, le mélange de solvants étant constitué d'eau et d'un solvant organique miscible à l'eau, contenant au moins deux atomes d'oxygène et présentant une constante diélectrique de 50 à 78,
c) la phase dispersée contient au moins une oléfine et
d) le facteur de charge du réacteur tubulaire est égal ou supérieur à 0,8.

**2.** Procédé selon la revendication 1, **caractérisé en ce que** le catalyseur contient un métal du 8ème groupe secondaire du système périodique des éléments.

**3.** Procédé selon la revendication 1 ou 2, **caractérisé en ce que** le catalyseur contient du rhodium.

**4.** Procédé selon l'une quelconque des revendications 1 à 3, **caractérisé en ce qu'**on utilise, comme catalyseur, des composés du rhodium solubles dans l'eau.

**5.** Procédé selon l'une quelconque des revendications 1 à 4, **caractérisé en ce que** le facteur de charge B est supérieur ou égal à 0,9.

**6.** Procédé selon l'une quelconque des revendications 1 à 5, **caractérisé en ce que** le facteur de charge B est supérieur ou égal à 1,0.

**7.** Procédé selon l'une quelconque des revendications 1 à 6, **caractérisé en ce que** le rapport massique de la phase continue à la ou aux phases dispersées est supérieur à 2.

**8.** Procédé selon l'une quelconque des revendications 1 à 7, **caractérisé en ce que** la phase continue entraîne un pulvérisateur en amont du réacteur tubulaire.

**9.** Procédé selon l'une quelconque des revendications 1 à 8, **caractérisé en ce qu'**au moins un produit de départ est dispersé par l'énergie introduite par la phase continue dans le réacteur tubulaire.

# Fig.1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3234701
- DE 2715685
- EP 0157316 A
- DE 19700805 C1
- DE 19700804 C1
- DE 19925384

**In der Beschreibung aufgeführte Nicht-Patentliteratur**

- **B. CORNILS ; W. A HERRMANN.** Aqueous-Phase Organometallic Catalysis. Wiley-VCH, 316-317
- Recent Advances in Basic and Applied Aspects of Industrial Catalysis. **V. S. R. NAIR ; B. M. BHANAGE ; R.M. DESHPANDE ; R.V. CHAUDHARI.** Studies in Surface Science and Catalysis. Elevier Science B.V, 1998, vol. 113, 529-539
- **S. ERGUN.** *Chem. Engng. Progr.,* 1948, vol. 48, 89
- **Y. SATO ; T.HIROSE ; F. TAKAHASHI ; M. TODA.** Pressure Loss and Liquid Hold Up in Packed Bed Reactor with Cocurrent Gas-Liquid Down Flow. *J. Chem. Eng. Of Japan,* 1973, vol. 6 (2), 147-152
- **D. SWEENEY.** A Correlation for Pressure Drop in Two-Phase Concurrent Flow in Packed Beds. *AlChE-Journal,* Juli 1967, vol. 13, 663-669
- **V. W. WEEKMAN ; J. E. MYERS.** Fluid-Flow Characteristics of Concurrent Gas-Liquid Flow in Packed Beds. *AIChE-Journal,* November 1964, vol. 10 (6), 951-957
- **R. P. LARKINS ; R P. WHITE ; D. W. JEFFREY.** Two-Phase Concurrent Flow in Packed Beds. *AlChE-Journal,* vol. 7 (2), 231-239
- **N. MIDOUX ; M. FAVIER ; J.- C. CHARPENTIER.** Flow Pattern, Pressure Loss and Liquid Holdup Data in Gas-Liquid Down-Flow Packed Beds with Foaming and Nonfoaming Liquids. *J. Chem. Eng. Of Japan,* 1976, vol. 9 (5), 350-356